# EUROPEAN PATENT SPECIFICATION

(11) **EP 2 645 960 B1**
(45) Date of publication and mention of the grant of the patent: **08.07.2015**
(21) Application number: 11788316.5
(22) Date of filing: 18.11.2011
(51) Int. Cl.: A61F 2/00

(54) **CUSHIONED RESILIENT INTRAVAGINAL UNINARY INCONTINENCE DEVICE**
ELASTISCHE GEPOLSTERTE INTRAVAGINALE HARNINKONTINENZVORRICHTUNG
DISPOSITIF D'INCONTINENCE URINAIRE INTRAVAGINAL ÉLASTIQUE AMORTI

(30) Priority: 30.11.2010 US 956824
(43) Date of publication of application: 09.10.2013
(73) Proprietor: First Quality Hygienic, Inc., Great Neck, NY 11021 (US)
(72) Inventor: MAVINKURVE, Pramod, Princeton, NJ 08540 (US); HOU, Mari, Basking Ridge, NJ 07920 (US); HULL, Raymond, J., Jr., Hampton, NJ 08827 (US); ROSENFELD, Leonard, Yardley, PA 19067 (US)
(74) Representative: Kirsch, Susan Edith
(86) International application number: PCT/US2011/061314
(87) International publication number: WO 2012/074779

(56) References cited:
- WO-A2-2009/044394
- US-A- 1 926 518
- US-A1- 2008 033 230

## Description

### Field of the invention

The present invention relates to an intravaginal urinary incontinence device. More specifically, this invention relates to a device that has a working portion and an anchoring portion and is overmolded with a cushioning material. The device is very useful for reducing or preventing urinary incontinence, and the overmolded cushioning material reduces the potential for vaginal irritation by reducing the pressure applied by the device on the vaginal wall during insertion, use or removal.

### Description of the prior art

Stress urinary incontinence is a problem for many women. It is characterized by leakage of urine during a stressing event, such as a cough or a sneeze. Many devices have been designed to reduce or prevent stress urinary incontinence. Tutrone, Jr., US Pat. No. 5,603,685 relates to inflatable devices and a means to provide a device that is small for insertion into the vagina and enlarges to a required shape and pressure to reduce or prevent urinary incontinence. Zunker et al., US Pat. No. 6,090,098 relates to tampon-like devices, each made with a combination of absorbing and/or non-absorbing fibrous materials. Ulmsten et al., US Pat. No. 6,645,137 relates to a coil that expands in the vagina to support the urinary system. Biswas, US Pat. No. 5,036,867 relates to a compressible resilient pessary. James, US Pat. No. 6,460,542 relates to a specifically shaped rigid pessary.

More recent developments have attempted to provide stent-like supports for deployment into the vagina. For example, Bartning et al., US Pat. App. Nos. 2008/0033230 and 2008/0009662 relate to an intravaginal urinary incontinence device that has an anchoring portion and a working portion. These documents also disclose covering the structure with a biocompatible material. In addition, there are numerous patents that relate to the use of small, appropriately-sized stents that are designed to keep body passages.

Sinai et al., US Pat. App. No. 2008/0281149, relates to an incontinence device with an internal and/or external resilient support member that biases arms of the incontinence device.

Finally, Ziv et al., WO2008/010214, relates to an intravaginal apparatus for treating urinary incontinence having a node connecting a support section and an anchoring section. This discloses the use of arms for the support and/or anchoring section made of silicone, nylon, polyurethane, foam polystyrene, metal, or an over molding of two materials.

Several of these references have begun to recognize the potential for the support structures to irritate vaginal tissues, and they have enclosed structural elements in tubing or other outer layers. Alternatively or in addition to this, bag-like covers have been suggested. Unfortunately, these developments have not fully addressed all of the issues relating to cushioning, comfort and product reliability.

Therefore, there are continuing needs for improved intravaginal urinary incontinence devices that can effectively reduce or prevent urinary incontinence on the one hand and can also provide appropriately located cushioning to avoid increased risk of vaginal damage.

### Summary of the Invention

We have found intravaginal urinary incontinence devices that have improved cushioning for comfort and safety in use.

In one embodiment of the invention, an intravaginal device has an insertion end, a withdrawal end, a longitudinal axis, and a device interior, and it includes a first structural material formed into a device frame and a second cushioning material having a thickness surrounding portions of the device frame. The first structural material includes a working portion disposed proximate the withdrawal end and an anchoring portion extending therefrom disposed proximate the insertion end. The device frame includes interconnected elongate elements having a maximum cross-section of less than about 5 mm and a longitudinal axis. The device frame has a plurality of primary bearing surfaces arranged and configured to bear on vaginal tissue during insertion and use, and the thickness of the cushioning material on the device frame is greater on the primary bearing surfaces than on surfaces of the device frame disposed toward the device interior. The device further includes a withdrawal element operatively connected to the working portion. Preferably, the first structural material is a high modulus polymer and the second material is a thermoplastic elastomer.

In another embodiment of the invention, an intravaginal device delivery system includes an intravaginal device contained in a delivery applicator. The intravaginal device has an insertion end, a withdrawal end, a longitudinal axis, and a device interior, and it includes a first structural material formed into a device frame and a second cushioning material having a thickness surrounding portions of the device frame. The first structural material includes a working portion disposed proximate the withdrawal end and an anchoring portion extending therefrom disposed proximate the insertion end. The device frame includes interconnected elongate elements having a maximum cross-section of less than about 5 mm and a longitudinal axis. The device frame has a plurality of bearing surfaces arranged and configured to bear on vaginal tissue during insertion and use. The thickness of the cushioning material on the device frame is greater on the primary bearing surfaces than on surfaces of the device frame disposed toward the device interior. The delivery applicator has an interior surface and a maximum internal diameter of less than about 24 mm. The device further includes a withdrawal element operatively connected to the working portion. Preferably, the first structural material is a high modulus polymer and the second material is a thermoplastic elastomer.

### Brief Description of the Drawing

Fig. 1 is a perspective view of an overmolded intravaginal incontinence device according to one aspect of the present invention.
Fig. 2 is a front view of the device of Fig. 1.
Fig. 3 is a perspective view of the device frame of the intravaginal incontinence device of Fig. 1.
Fig. 4 is a front view of the device frame of Fig. 3.
Fig. 5 is a side elevation of the device frame of Fig. 3.
Fig. 6 is a top view of the device frame of Fig. 3.
Fig. 7 is a front view of the intravaginal incontinence device of Fig. 1 contained within a flexible bag.
Fig. 8 is a perspective view of the intravaginal incontinence device of Fig. 1 contained within a delivery applicator.
Figs. 9A and 9B are cross-sections of mold elements for manufacturing the intravaginal incontinence device of Fig. 1.

### Detailed Description of the Preferred Embodiments

We have discovered that the descriptions of how to protect structural elements of intravaginal incontinence devices with a softer material disclosed in the art fail to show how to manufacture commercial quantities of inexpensive devices with adequate comfort for the user. First, we have not found overmolding processes with adequate control of the process to provide cushioning where needed without creating unnecessary bulk. Unnecessary bulk can make it difficult and/or impossible to provide a small enough applicator for the intravaginal incontinence device for comfortable insertion into the vagina with enough expansion to provide necessary support to an associated urinary system.

During the development of this invention, we have also discovered that low-cost injection molded structural elements in intravaginal incontinence devices can have a rough edge or part line at the periphery of the mold portions. This has the potential to irritate the vagina. Covering this device in a bag did not adequately address this problem, as these rough edges simply tore the bag during packaging of the product into an applicator and/or during the expulsion of the device to deploy it into a vagina.

Therefore, we have developed a partially overmolded device to answer some of these problems. This partial overmolded material can be non-uniform about the structural element that it covers. For example, the overmold material can be biased in a manner that the structural element is not located in the center of the overmold material. This will be discussed in greater detail, below.

It will be recognized that overmolding the structural elements of an intravaginal incontinence device increases the contact area between the device and the user's body tissue that it may engage, reducing the pressure (force per unit area). This helps to reduce or minimize vaginal irritation during insertion, use or removal.

The intravaginal incontinence devices of the present invention have a working portion to provide support to an associated urinary system and an anchoring portion to hold the device in optimal position during use. These structural elements are additionally covered, in appropriate locations, to cushion the body from irritation.

As used herein the specification and the claims, the term "stent" and variants thereof relate to a device used to support a bodily orifice, cavity, vessel, and the like. The stent is resilient, flexible, and collapsible with memory. The stent may be any suitable form, including, but not limited to, scaffolding, a slotted tube or a wire form.

As used herein the specification and the claims, the term "wire form" and variants thereof relate to a structure formed of at least one wire or wire-like material that is manipulated and optionally secured (e.g., by welding and/or molding) in a desired three-dimensional structure.

As used herein, the term "bearing surface" and variants thereof relate to certain portions of the device that bear on and apply pressure to the vaginal epithelium during the insertion, use and removal. The existence of bearing surfaces is significant, because poorly designed devices may have dangerous bearing surfaces that can damage the vagina and/or surrounding body tissue. This damage could include irritation, erythema, and weakened or even necrotic vaginal tissue. Therefore, it is critical to protect the vaginal epithelium by cushioning actual and potential bearing surfaces.

As used herein, the term "primary bearing surface" and variants thereof relate to certain portions of the bearing surfaces that exert the greatest pressure to the vaginal epithelium during the insertion, use and removal. In particular, these primary bearing surfaces include the leading surfaces during insertion, the leading surfaces during withdrawal and the surfaces of the anchoring portion that are biased away from the device interior (as defined below) and bear on the vaginal epithelium to maintain the device in its proper position during use, and the surfaces of the anchoring portion that are biased away from the device interior and bear on the vaginal epithelium to provide direct support to the urethra and/or bladder neck.

As used herein, the term "device interior" and variants thereof relate to the inner portions of the device, directed toward a longitudinal axis and away from the bearing surfaces that are capable of contacting the vaginal epithelium. The device interior also will be described with reference to the figures, below.

As used herein, the term "overmolding" and variants thereof relate to injection molding processes where the cushioning material is molded onto the device frame (i.e. the wire or stent). The overmolding is performed in such a manner that the cushioning material fully encapsulates the device frame at that location. The use of primers or adhesives is not required to achieve an optimum bond between the device frame or structural elements and the overlying cushioning material.

As used herein, the term "cushioning material" and variants thereof relate to any material which is soft in nature, the cushioning portion of the device provides softness and comfort and helps to reduce or minimize vaginal irritation and pressure mounted by the device on the vaginal epithelium during insertion, use or removal.

The intravaginal incontinence devices of the present invention include a working portion and anchoring portion. These portions are the structural elements of the device (also referenced as the "device frame"). The working portion provides support to an associated urinary system, and the anchoring portion maintains the working portion in an optimal location for this support. The overlying cushioning material provides comfort to the user. It can both smooth out any rough edges resulting from parting lines in the mold that formed the device frame, and it can increase the surface area over which the device contacts the user's body tissue to reduce the pressure.

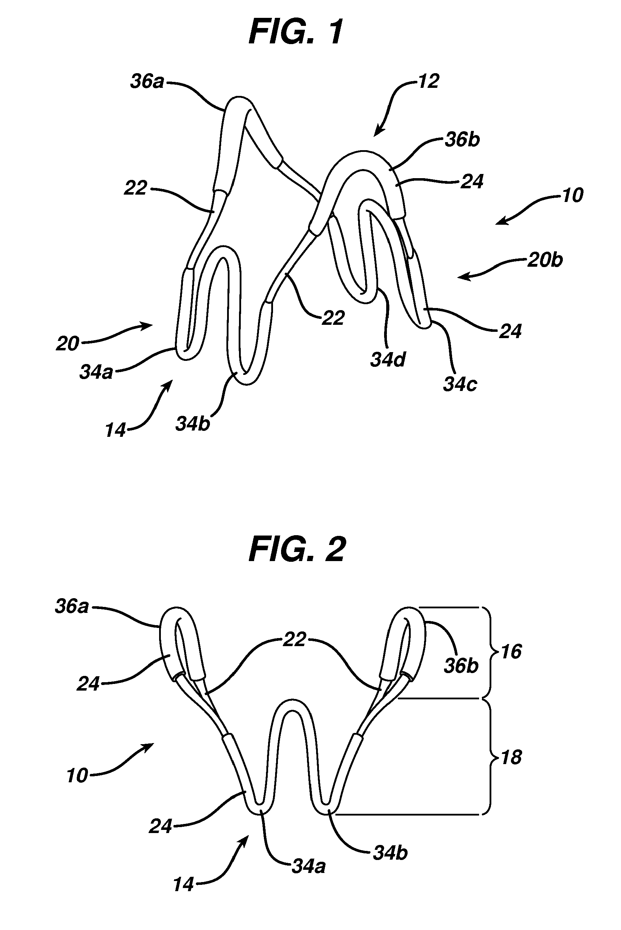

Suitable shapes of devices according to the present invention are taught in US Pat. App. Nos. 2008/0009664, and 2008/0033230, and 2008/0009662. Referring to Figs. 1-2, there is shown a device 10 according to the present invention. The device 10 has an insertion end 12, a withdrawal end 14, and a device interior 15. An anchoring portion 16 is disposed proximate the insertion end 12, and a working portion 18 is disposed proximate the withdrawal end 14. The working portion 18 has opposed faces 20a and 20b.

Working portion 18 includes a device frame 22 formed of a first structural material that provides resistance to compression and recovers from compression with sufficient force to provide the desired incontinence support. Useful structural materials are elastic or even superelastic materials. These structural materials include metals (including without limitation metal alloys), polymers (including without limitation shape memory polymers and high modulus polymers), composites of one or more polymers and/or filled or reinforced polymers, and combinations thereof. Shape memory materials include those disclosed in US Pat. App. Nos. 2008/0009664, and 2008/0033230, and 2008/0009662. High modulus polymers include those disclosed in copending application, Serial No. 12/645800, filed on December 23, 2009, entitled "Intravaginal Incontinence Device."

Preferred high modulus polymers have an elongation at yield of at least 3% and an elastic modulus of at least 2 Gpa. A representative, non-limiting list of suitable high modulus polymers includes polyetherimide, polyetheretherketone, polycarbonate, co-polymers, specialized and/or modified plastics, filled plastics, and the like, that can provide these high modulus properties. Preferred high modulus polymers include polyetherimides and polyetheretherketones. These materials are further described in the above-mentioned copending application, Serial No. 12/645800, filed on December 23, 2009.

Portions of the device frame 22 are overmolded with a cushioning material 24, and the amount of cushioning material is greater in areas of the device that create bearing surfaces during insertion, use or removal of the devices.

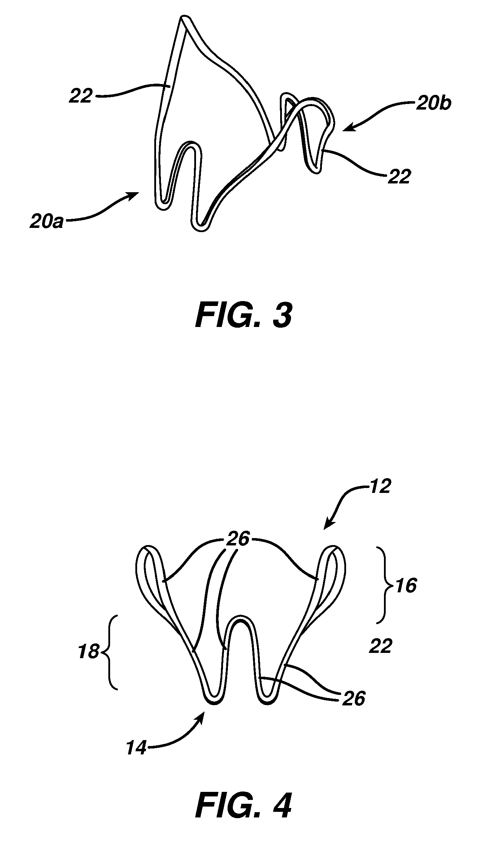

Figs. 3-6 show the device frame 22 without the cushioning material. The working portion 18 of the device frame 22 is formed of a plurality of connected elongate elements 26. The elongate elements 26 that make up the working portion may directly or indirectly connect to those elongate elements 26 that make up the anchoring portion 16. The working pressure exerted by the working portion 18 is determined by the material selected for the device frame 22 and by the dimensions and arrangement of the elongate elements 26 that make up this device frame 22. Thicker elongate elements and/or shorter elongate elements can generally provide greater working pressures as these are capable of providing greater resistance to deformation of the device and, thus, greater expansion force when the device is compressed or reduced in cross-section. In addition, the angle between the elongate elements also influences the working pressure.

The elongate elements 26 have a small cross-section in order to fit into a delivery applicator and to be comfortable for the user. The elongate elements should have a maximum cross-section of less than about 5 mm, preferably, less than about 4 mm, and most preferably, less than about 3 mm. The elongate elements 26 can have any useful cross-section shape, including without limitation, round, oval, elliptical, triangular, rectangular, etc. As one of ordinary skill will recognize, the change cross-section shape may provide various desired resilience, increased surface area for a given cross-sectional area, reduced material stress, and the like.

Anchoring portion may be formed of the same materials as the working portion, and in a preferred embodiment, both the working portion and the anchoring portion are formed of the same material in a unitary construction.

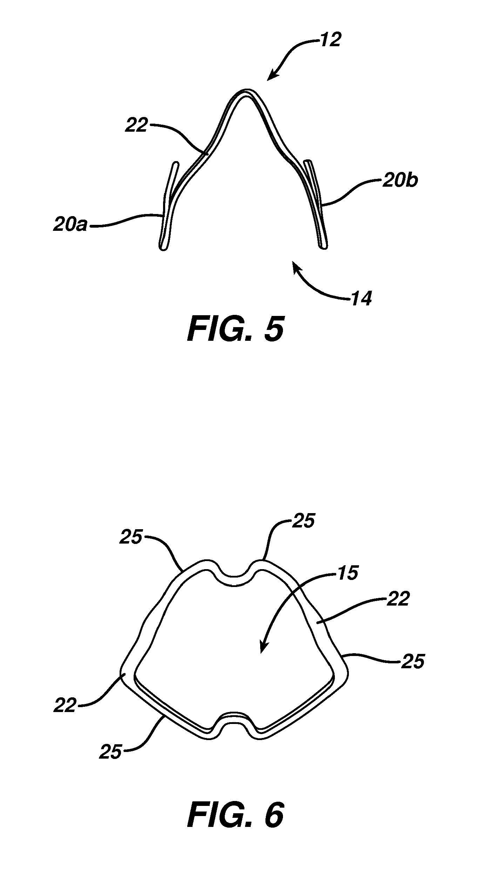

As shown in Fig. 6, a top view of the device frame 22, the device interior 15 is preferably open, and the device frame 22 loosely defines this cavity or hollow. The bearing surfaces are generally disposed on outwardly-facing surfaces 25 of the device frame 22.

As discussed in greater detail, below, the bearing surfaces that may apply the greatest forces to the vaginal epithelium during insertion, use, and withdrawal of the device, are preferentially overmolded with the cushioning material 24 (as shown in Figs. 1 and 2). This provides useful characteristics to the device. The cushioning material may provide one or more of the following properties to the intravaginal incontinence device: resiliency, shock absorbing, softness, elasticity, tear-resistance, protection of the frame from chemical degradation (for example, by oxidation or other chemical attack, especially in high stress portions), and the like. In addition, the cushioning material may provide other functions including acting as a carrier for medications, lotions, fragrances, odor neutralizers, lubricants, and the like. The cushioning material can also improve the aesthetics of the device, especially if the incontinence device is visible, and it can improve the ability of the device to stay in place by providing a textured and/or a more compliant surface.

The properties such as resiliency, shock absorbing, softness, elasticity, flexibility, and the like can provide the softness and cushioning to minimize excessive pressure on the vaginal tissues. Properties, such as elasticity and tear-resistance can provide additional safety in the event of breakage of the device frame. The cushioning material can act to contain such broken elements. In addition, the relatively soft, elastic, and/or flexible materials provide decrease the likelihood that parting lines from the molded part are sharp enough to be a source for irritation of vaginal tissues during insertion, use, and withdrawal of the device.

The cushioning material may be formed of any soft and/or flexible material useful in injection molding and/or dip molding processes that providing desired properties, such as thermoplastic elastomers. Useful materials for the cushioning material include, without limitation, urethanes, polyolefins (including polyethylenes, polypropylenes, ethylene-propylene diene monomers, etc.), co-polymers (including styrene-ethylene-butylene-styrene block co-polymers such as the KRATON® thermoplastic elastomers from Kraton Polymers), styrene acrylate co-polymers, silicones, rubber, latex, fibers, and the like. In addition, mixtures and blends of materials can also be used including, without limitation, Santoprene™ thermoplastic elastomer from ExxonMobil Chemical.

One measure of the appropriateness of the cushioning material is a measure of the Shore A Hardness. Preferably, the cushioning material has a Shore A Hardness of between about 0 to about 120, preferably in a range of about 20 to about 100, more preferably in a range of about 40 to about 90 Shore A Hardness.

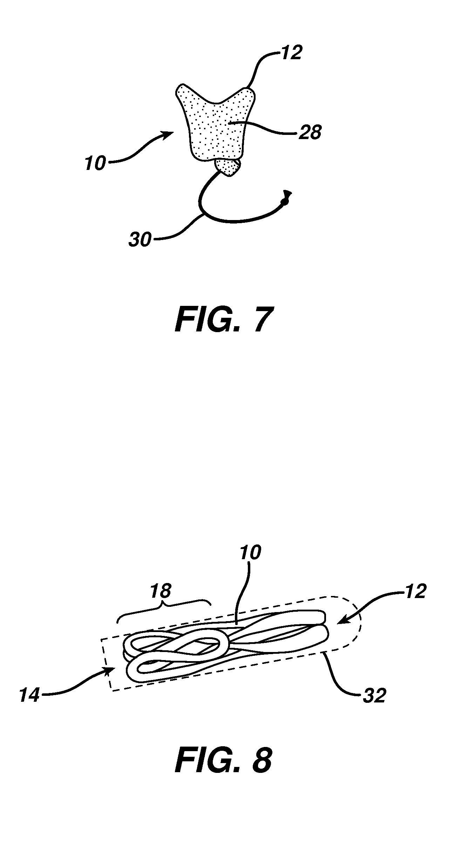

As shown in Fig. 7, the device 10 may also be enclosed in a flexible bag 28 or other relatively loose cover. This bag may provide one or more beneficial properties. It may reduce friction between the intravaginal incontinence device and its applicator and/or vaginal tissue during deployment. The flexible bag 28 may hide or otherwise disguise the appearance of the device frame from view for a more acceptable consumer device. The flexible bag 28 may help control the device during insertion and removal. It may help the device to stay in place. The flexible bag 28 may also contain other optional components such as a suppository substance. Finally, the flexible bag 28 may increase the contact area for applying pressure to the bladder neck. The cover may also provide increased friction against the vaginal epithelium to help the device stay in place during use. Any medically appropriate materials may be used to form the bag, and depending upon the desired end-use, it may be opaque, light, and/or breathable. Useful bag materials include those used in the manufacture of tampons, such as nonwoven fabrics and plastic film, including apertured films. The bag itself may also be apertured.

The intravaginal incontinence device preferably includes a withdrawal element such as a removal string 30. This may be crisscrossed between the elongate elements of the device frame to create a "cinch sac" mechanism. Any string or cord known in the sanitary protection art may be useful for this purpose. As the strings are pulled during removal, the elongate elements are gathered together to create a smaller diameter device during removal. Cinching the device at its base may make removal of the device more comfortable and easier as it makes the diameter of the device smaller and the shape conducive to remove easily.

The intravaginal incontinence device may be contained within an applicator 32 (shown in phantom in Fig. 8) similar to those known for use in delivering tampons and suppositories. The applicator may be a push-type applicator or a retractable applicator. Preferred delivery applicators have a maximum internal diameter of less than about 24 mm, more preferably, less than about 19 mm, and most preferably less than 16 mm. A collar may be added to control the depth of insertion.

In a preferred embodiment, the cushioning material is non-uniform about the device frame 22 that it covers. We have found that biasing the cushioning material to the outer surfaces of the device frame provides more useful cushioning while minimizing the volume of the cushioning material that merely adds bulk to the device.

Referring again to Figs. 1 and 2, device 10 is overmolded with a cushioning material 24 at the contact areas between the device 10 and the vaginal epithelium in use. As seen in Fig. 1, the primary bearing surfaces of the device 10, those that bear on the vaginal epithelium with the greatest pressure, may be located e.g., at the bottom elongate element intersections 34a-34d in the "W-shaped" section of each face 20a and 20b of the working portion 18, and at the top bends 36a and 36b of the "ear section" of the anchoring portion 16. These primary bearing surfaces may apply the greatest forces to the vaginal epithelium during insertion, use, and withdrawal of the device, and they are preferentially overmolded with the cushioning material 24.

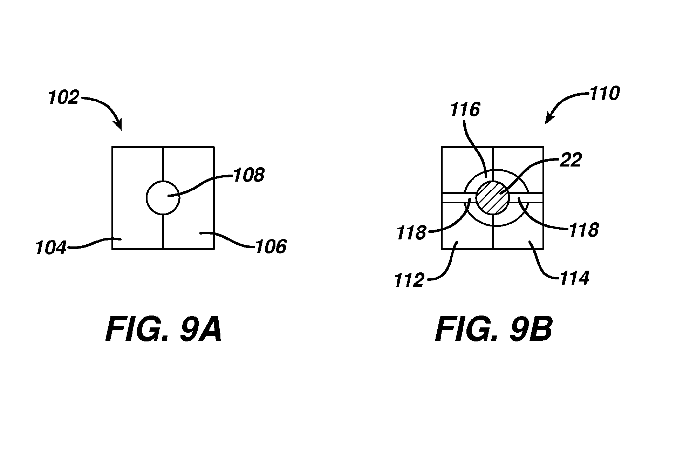

As seen in Figs. 9A-9B, discussed below, the cushioning is performed in a manner that provides more thickness at the primary bearing surfaces of the device 20 and reduced thickness of the cushioning material 24 directed toward the device interior 15. Preferably, the thickness ratio of the cushioning material (thickness on primary bearing surface : to thickness on the opposite surface of the elongate element, directed toward the device interior) is at least 1.1:1. More preferably, the thickness ratio of the cushioning material is at least 1.5:1, and most preferably, it is at least 2:1.

As seen in Figs. 9A-9B, in one embodiment of the invention the device 10 is overmolded by two step process. A plastic device frame is formed in a first mold element 102 having first mold half 104 and second mold half 106 and providing a first mold cavity 108. The resulting molded device frame 22 (such as shown in Figs. 3-5) is transferred to a second mold element 110 having first mold half 112 and second mold half 114 and providing a second mold cavity 116. Portions of the second mold element 110 are dimensioned to securely hold the device frame 22 to secure it in place and provide no void volume in the mold cavity. These portions are not overmolded with cushioning material and correspond to the uncovered portions of the device 10 shown in Figs. 1 and 2. The portions of the device frame that are to be overmolded are located in the second mold cavity 116 having void volume for overmolding about the device frame 22. These portions of the device frame 22 are maintained in position by centering pins 118. These centering pins 118 function to stabilize the device frame 22 from deflection as the cushioning material is injected into the second mold cavity 116. After the overmolding is complete, the mold is opened, the pins may be retracted as needed, and the resulting overmolded device is ejected from the second mold cavity 116.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. An intravaginal device (10) having an insertion end (12), a withdrawal end (14), a longitudinal axis, and a device interior (15), the device comprising
a. a first structural material formed into a device frame (22) comprising a working portion (18) disposed proximate the withdrawal end and an anchoring portion (16) extending therefrom disposed proximate the insertion end, the device frame comprising interconnected elongate elements (26) having a maximum cross-section of less than about 5 mm;
b. a second cushioning material (24) having a thickness surrounding portions of the device frame;
wherein the device frame has a plurality of primary bearing surfaces (36a, 36b, 34a - 34d) arranged and configured to bear on vaginal tissue during insertion and use, and wherein a withdrawal element (30) is operatively connected to the working portion,
**characterised in that** the thickness of the cushioning material on the device frame is greater on the primary bearing surfaces than on surfaces of the device frame disposed toward the device interior.

2. The intravaginal device (10) of claim 1, wherein the elongate elements (26) of the device frame have a generally circular cross-section.

3. The intravaginal device (10) of claim 1, wherein the elongate elements (26) of the device frame have a variable cross-section.

4. The intravaginal device (10) of claim 3, wherein at least some of the elongate elements (26) of the anchoring portion have a non-circular cross-section.

5. The intravaginal device (10) of claim 4, wherein the anchoring portion (16) has elongate elements (26) at the insertion end having a generally oval cross-section with a major axis of this oval cross section substantially perpendicular to the longitudinal axis of the device.

6. The intravaginal device (10) of claim 1, wherein the first structural material comprises a composite material.

7. The intravaginal device (10) of claim 1, wherein the second cushioning material (24) comprises a thermoplastic elastomer.

8. The intravaginal device (10) of claim 1, wherein the second cushioning material (24) comprises polymeric material having a Shore A Hardness of between about 0 to about 120.

9. The intravaginal device (10) of claim 8, wherein the second cushioning material (24) comprises polymeric material having a Shore A Hardness of between about 40 to about 90.

10. An intravaginal device delivery system comprising:
a. an intravaginal device (10) having an insertion end (12), a withdrawal end (14), a longitudinal axis, and a device interior (15) and comprising:
i. a first structural material formed into a device frame (22) consisting a working portion (18) disposed proximate the withdrawal end and an anchoring portion (16) extending therefrom disposed proximate the insertion end, the device frame comprising interconnected elongate elements (26) having a maximum cross-section of less than about 5 mm and a longitudinal axis;
ii. a second cushioning material (24) having a thickness surrounding portions of the device frame;
wherein the device frame has a plurality of primary bearing surfaces (36a, 36b, 34a - 34d) arranged and configured to bear on vaginal tissue during insertion and use, and wherein a withdrawal element (30) is operatively connected to the working portion;
b. a delivery applicator (32) arranged and configured to contain the intravaginal device (10), wherein the delivery applicator has an interior surface and a maximum internal diameter of less than about 24mm.
**characterised in that** the thickness of the cushioning material on the device frame of the intravaginal device is greater on the primary bearing surfaces than on surfaces of the device frame disposed toward the device interior.

11. The intravaginal device delivery system of claim 10, wherein the elongate elements of the device frame (22) have a variable cross-section and wherein at least some of the elongate elements of the anchoring portion (16) have a non-circular cross-section.

12. The intravaginal device (10) of claim 11, wherein the anchoring portion (16) has elongate elements at the insertion end (12) having a generally oval cross-section with a major axis of this oval cross section substantially perpendicular to the longitudinal axis of the device.

13. The intravaginal device (10) of claim 1 or 10, wherein the first structural material comprises a high modulus polymeric material having an elongation at yield of at least 3% and an elastic modulus of at least 2 Gpa.

14. The intravaginal device (10) of claim 10, wherein the second cushioning material (24) comprises a thermoplastic elastomer having a Shore A Hardness of between about 0 to about 120.

15. The intravaginal device (10) of claim 1 or 10, wherein the withdrawal element (30) is directly connected to the working portion.

## Patentansprüche

1. Intravaginale Vorrichtung (10) mit einem Einführungsende (12), einen Entnahmeende (14), einer Längsachse und einem Vorrichtungsinnenraum (15), wobei die Vorrichtung Folgendes umfasst:
a. ein erstes Strukturmaterial, das in einen Vorrichtungsrahmen (22) geformt ist, der einen Arbeitsabschnitt (18) in der Nähe des Entnahmeendes und einen Verankerungsabschnitt (16), der sich davon erstreckt und in der Nähe des Einführungsendes angeordnet ist, umfasst, wobei der Vorrichtungsrahmen miteinander verbundene langgestreckte Elemente (26) mit einem maximalen Querschnitt von weniger als ungefähr 5 mm umfasst;
b. ein zweites Polstermaterial (24) mit einer Abschnitte des Vorrichtungsrahmens umgebenden Dicke;
worin der Vorrichtungsrahmen eine Vielzahl von primären Anlageflächen (36a, 36b, 34a-34d) aufweist, die angeordnet und ausgelegt sind, während der Einführung und Verwendung an Vaginalgewebe anzuliegen, und worin ein Rückziehelement (30) mit dem Arbeitsabschnitt in Wirkverbindung steht,
**dadurch gekennzeichnet, dass** die Dicke des Polstermaterials auf dem Vorrichtungsrahmen auf den primären Anlageflächen größer ist als auf Flächen des Vorrichtungsrahmens, die zum Vorrichtungsinnenraum hin angeordnet sind.

2. Intravaginale Vorrichtung (10) nach Anspruch 1, worin die langgestreckten Elemente (26) des Vorrichtungsrahmens einen allgemein kreisförmigen Querschnitt aufweisen.

3. Intravaginale Vorrichtung (10) nach Anspruch 1, worin die langgestreckten Elemente (26) des Vorrichtungsrahmens einen variablen Querschnitt aufweisen.

4. Intravaginale Vorrichtung (10) nach Anspruch 3, worin zumindest einige der langgestreckten Elemente (26) des Verankerungsabschnitts einen nicht-kreisförmigen Querschnitt aufweisen.

5. Intravaginale Vorrichtung (10) nach Anspruch 4, worin der Verankerungsabschnitt (16) an dem Einführungsende langgestreckte Elemente (26) aufweist, die einen allgemein ovalen Querschnitt aufweisen, wobei eine Hauptachse dieses ovalen Querschnitts im Wesentlichen senkrecht zu der Längsachse der Vorrichtung ist.

6. Intravaginale Vorrichtung (10) nach Anspruch 1, worin das erste Strukturmaterial ein Verbundmaterial umfasst.

7. Intravaginale Vorrichtung (10) nach Anspruch 1, worin das zweite Polstermaterial (24) ein thermoplastisches Elastomer umfasst.

8. Intravaginale Vorrichtung (10) nach Anspruch 1, worin das zweite Polstermaterial (24) ein Polymermaterial mit einer Shore-A-Härte zwischen ungefähr 0 und ungefähr 120 umfasst.

9. Intravaginale Vorrichtung (10) nach Anspruch 8, worin das zweite Polstermaterial (24) ein Polymermaterial mit einer Shore-A-Härte zwischen ungefähr 40 und ungefähr 90 umfasst.

10. Einführungssystem für eine intravaginale Vorrichtung, umfassend:
a. eine intravaginale Vorrichtung (10) mit einem Einführungsende (12), einem Entnahmeende (14), einer Längsachse und einem Vorrichtungsinnenraum (15), und umfassend:
i. ein erstes Strukturmaterial, das in einen Vorrichtungsrahmen (22) geformt ist, der einen Arbeitsabschnitt (18) in der Nähe des Entnahmeendes und einen Verankerungsabschnitt (16), der sich davon erstreckt und in der Nähe des Einführungsendes angeordnet ist, umfasst, wobei der Vorrichtungsrahmen miteinander verbundene langgestreckte Elemente (26) mit einem maximalen Querschnitt von weniger als ungefähr 5 mm und eine Längsachse umfasst;
ii. ein zweites Polstermaterial (24) mit einer Abschnitte des Vorrichtungsrahmens umgebenden Dicke;
worin der Vorrichtungsrahmen eine Vielzahl von primären Anlageflächen (36a, 36b, 34a-34d) aufweist, die angeordnet und ausgelegt sind, während der Einführung und Verwendung an Vaginalgewebe anzuliegen, und worin ein Rückziehelement (30) mit dem Arbeitsabschnitt in Wirkverbindung steht;
b. einen Einführungsapplikator (32), der angeordnet und ausgelegt ist, die intravaginale Vorrichtung (10) zu enthalten, worin der Einführungsapplikator eine Innenfläche und einen maximalen Innendurchmesser von weniger als ungefähr 24 mm aufweist,
**dadurch gekennzeichnet, dass** die Dicke des Polstermaterials auf dem Vorrichtungsrahmen der intravaginalen Vorrichtung auf den primären Anlageflächen größer ist als auf Flächen des Vorrichtungsrahmens, die zum Vorrichtungsinnenraum hin angeordnet sind.

11. Einführungssystem für eine intravaginale Vorrichtung nach Anspruch 10, worin die langgestreckten Elemente des Vorrichtungsrahmens (22) einen variablen Querschnitt aufweisen und worin zumindest einige der langgestreckten Elemente des Verankerungsabschnitts (16) einen nicht-kreisförmigen Querschnitt aufweisen.

12. Intravaginale Vorrichtung (10) nach Anspruch 11, worin der Verankerungsabschnitt (16) an dem Einführungsende (12) langgestreckte Elemente aufweist, die einen allgemein ovalen Querschnitt aufweisen, wobei eine Hauptachse dieses ovalen Querschnitts im Wesentlichen senkrecht zu der Längsachse der Vorrichtung ist.

13. Intravaginale Vorrichtung (10) nach Anspruch 1 oder 10, worin das erste Strukturmaterial ein Hochmodulpolymermaterial mit einer Streckdehnung von mindestens 3% und einem Elastizitätsmodul von mindestens 2 Gpa umfasst.

14. Intravaginale Vorrichtung (10) nach Anspruch 10, worin das zweite Polstermaterial (24) ein thermoplastisches Polymer mit einer Shore-A-Härte zwischen ungefähr 0 und ungefähr 120 umfasst.

15. Intravaginale Vorrichtung (10) nach Anspruch 1 oder 10, worin das Rückziehelement (30) direkt mit dem Arbeitsabschnitt verbunden ist.

## Revendications

1. Dispositif intravaginal (10) présentant une extrémité d'insertion (12), une extrémité de retrait (14), un axe longitudinal, et un intérieur de dispositif (15), le dispositif comprenant :
a. un premier matériau structurel façonné à la forme d'un cadre de dispositif (22) comprenant une portion de travail (18) disposée à proximité de l'extrémité de retrait et une portion d'ancrage (16) s'étendant depuis celle-ci et disposée à proximité de l'extrémité d'insertion, le cadre de dispositif comprenant des éléments allongés interconnectés (26) ayant une section transversale maximale inférieure à environ 5 mm ;
b. un deuxième matériau d'amortissement (24) ayant une épaisseur entourant des portions du cadre de dispositif ;
le cadre de dispositif ayant une pluralité de surfaces d'appui primaires (36a, 36b, 34a-34d) disposées et configurées de manière à presser contre un tissu vaginal lors de l'insertion et de l'utilisation, et un élément de retrait (30) étant connecté fonctionnellement à la portion de travail,
**caractérisé en ce que** l'épaisseur du matériau d'amortissement sur le cadre de dispositif est plus importante sur les surfaces d'appui primaires que sur les surfaces du cadre de dispositif disposées vers l'intérieur du dispositif.

2. Dispositif intravaginal (10) selon la revendication 1, dans lequel les éléments allongés (26) du cadre de dispositif présentent une section transversale généralement circulaire.

3. Dispositif intravaginal (10) selon la revendication 1, dans lequel les éléments allongés (26) du cadre de dispositif présentent une section transversale variable.

4. Dispositif intravaginal (10) selon la revendication 3, dans lequel au moins certains des éléments allongés (26) de la portion d'ancrage présentent une section transversale non circulaire.

5. Dispositif intravaginal (10) selon la revendication 4, dans lequel la portion d'ancrage (16) présente des éléments allongés (26) au niveau de l'extrémité d'insertion, ayant une section transversale généralement ovale avec un axe principal de cette section transversale ovale substantiellement perpendiculaire à l'axe longitudinal du dispositif.

6. Dispositif intravaginal (10) selon la revendication 1, dans lequel le premier matériau structurel comprend un matériau composite.

7. Dispositif intravaginal (10) selon la revendication 1, dans lequel le deuxième matériau d'amortissement (24) comprend un élastomère thermoplastique.

8. Dispositif intravaginal (10) selon la revendication 1, dans lequel le deuxième matériau d'amortissement (24) comprend un matériau polymère ayant une dureté Shore A comprise entre environ 0 et environ 120.

9. Dispositif intravaginal (10) selon la revendication 8, dans lequel le deuxième matériau d'amortissement (24) comprend un matériau polymère ayant une dureté Shore A comprise entre environ 40 et environ 90.

10. Système de distribution de dispositif intravaginal comprenant :
a. un dispositif intravaginal (10) ayant une extrémité d'insertion (12), une extrémité de retrait (14), un axe longitudinal, et un intérieur de dispositif (15) et comprenant :
i. un premier matériau structurel façonné à la forme d'un cadre de dispositif (22) comprenant une portion de travail (18) disposée à proximité de l'extrémité de retrait et une portion d'ancrage (16) s'étendant depuis celle-ci et disposée à proximité de l'extrémité d'insertion, le cadre de dispositif comprenant des éléments allongés interconnectés (26) ayant une section transversale maximale inférieure à environ 5 mm et un axe longitudinal ;
ii. un deuxième matériau d'amortissement (24) ayant une épaisseur entourant des portions du cadre de dispositif ;
le cadre de dispositif ayant une pluralité de surfaces d'appui primaires (36a, 36b, 34a-34d) disposées et configurées de manière à presser contre un tissu vaginal lors de l'insertion et de l'utilisation, et un élément de retrait (30) étant connecté fonctionnellement à la portion de travail ;
b. un applicateur de distribution (32) prévu et configuré pour contenir le dispositif intravaginal (10), l'applicateur de distribution ayant une surface intérieure et un diamètre intérieur maximal inférieur à environ 24 mm **caractérisé en ce que** l'épaisseur du matériau d'amortissement sur le cadre de dispositif du dispositif intravaginal est plus importante sur les surfaces d'appui primaires que sur les surfaces du cadre de dispositif disposées vers l'intérieur du dispositif.

11. Système de distribution de dispositif intravaginal selon la revendication 10, dans lequel les éléments allongés du cadre de dispositif (22) présentent une section transversale variable et dans lequel certains au moins des éléments allongés de la portion d'ancrage (16) présentent une section transversale non circulaire.

12. Dispositif intravaginal (10) selon la revendication 11, dans lequel la portion d'ancrage (16) présente des éléments allongés au niveau de l'extrémité d'insertion (12), ayant une section transversale généralement ovale avec un axe principal de cette section transversale ovale substantiellement perpendiculaire à l'axe longitudinal du dispositif.

13. Dispositif intravaginal (10) selon la revendication 1 ou 10, dans lequel le premier matériau structurel comprend un matériau polymère à module élevé, ayant un allongement au seuil de fluage d'au moins 3 % et un module d'élasticité d'au moins 2 Gpa.

14. Dispositif intravaginal (10) selon la revendication 10, dans lequel le deuxième matériau d'amortissement (24) comprend un élastomère thermoplastique ayant une dureté Shore A comprise entre environ 0 et environ 120.

15. Dispositif intravaginal selon la revendication 1 ou 10, dans lequel l'élément de retrait (30) est connecté directement à la portion de travail.
